# EUROPEAN PATENT SPECIFICATION

(11) **EP 2 967 914 B1**
(45) Date of publication and mention of the grant of the patent: **10.05.2017**
(21) Application number: 14711592.7
(22) Date of filing: 14.03.2014
(51) Int. Cl.: A61F 2/46, A61F 2/36

(54) **IMPLANT TRIALLING**
TESTEN VON IMPLANTATEN
EXPÉRIMENTATION D'IMPLANT

(30) Priority: 15.03.2013 GB 201304835
(43) Date of publication of application: 20.01.2016
(73) Proprietor: DePuy Ireland Unlimited Company, County Cork (IE)
(72) Inventor: HUNT, Christopher, Leeds LS11 0EA (GB); PRINCE, Stephanie, Leeds LS11 0EA (GB)
(74) Representative: Alton, Andrew
(86) International application number: PCT/GB2014/050816
(87) International publication number: WO 2014/140636

(56) References cited:
- WO-A1-2009/106867
- DE-A1- 10 335 442
- US-A- 5 888 211
- US-A1- 2004 199 257

## Description

The present invention relates to implant trialling and in particular to trial implants for ball and socket type joints.

Prosthetic implants can be used to replace ball and socket type joints such as shoulder joints and hip joints. Many of these prosthetic implants have the general construction of a stem component, which is received in a long bone (such as the femur or humerus) to which a head component is attached and which provides an articulating surface. The head component can be received in a component having a concave articulating surface, sometimes referred to as a cup, so as to reconstitute the full ball and socket type joint.

During the surgical procedure it is often important accurately to place and position the prosthetic components. Typically cavities are formed in the patient's bones, for example using a broach or reamer, and parts of the patient's bones are resected during the surgical procedure, for example using a saw, in order to prepare the bones for implantation of the prosthetic joint. The preparation of the bones is not perfectly reliable and reproducible and so sometimes the actual positioning of the implants in the patient's prepared bones does not correspond to a planned position. Therefore, as part of the surgical procedure, trial components are often used to help assess intra-operatively the position of the implants and determine whether any changes to the implants or the bone preparation may be needed to bring the actual implant position closer to the planned position.

Implants are often provided in a variety of different sizes and the most appropriate size for an individual patient may not become clear until intra-operatively, when the bones have been prepared. It can therefore be necessary to provide a wide range of sizes of trial components to cover most scenarios. Further, when it is possible to vary a property of the implant, for example the off set, by combining different parts, then a variety of different implant parts can be needed for each different implant size. This can give rise to a large number of trial parts which need to be provided which can lead to confusion, reduced efficiency of the surgical procedure, increased inventory and increased manufacturing difficulty and costs.

An example of a trial implant for a ball and socket type joint with a stem component and an adjustable articulating component is described in US-5,569,263. The articulating component is attached to a neck extending from a stem component and which has a plurality of grooves along its length. A locking mechanism can engage selectively with a one of the grooves to adjust the position of the articulating component relative to the stem to adjust the joint centre of rotation position.

Another adjustable trial implant us described in DE 102008030260. The trial implant has an articulating component which can detachably connected to a further component in different connecting positions relative to each other for example by using a screw thread mechanism.

US-5,888,211 describes an adaptor for a bipolar or unipolar head trial. The adaptor is mounted on a femoral neck trunnion and the adaptor is positively seated and retained by an annular shelf within a cavity of the head trial. An adjustable version of the adaptor includes a barrel passing through a cylindrical portion. The cylindrical portion is received within the mouth of the trial head. The barrel is mounted on the trunnion. The barrel includes three grooves corresponding to different neck length positions and which can be engaged by a pawl within the cylindrical portion to lock the barrel at a desired position relative to the cylindrical component corresponding to a desired neck length. Alternatively a plurality of fixed length adaptors can be used each representing different relative neck lengths.

Hence, it would be beneficial to make trialling for ball and socket type joints simpler.

According to a first aspect of the invention, there is provided a trial implant system comprising: a stem component having a neck including a free end, the neck having a neck longitudinal axis; a first trial head having an articulating surface, the first trial head including a bore having a bore longitudinal axis and which can receive the free end of the neck with the neck longitudinal axis and bore longitudinal axis coincident; a first formation; and a plurality of second formations, wherein one of the first formation and the plurality of second formations is provided on the neck and the other is provided within the bore and wherein each of the second formations can matingly engage with the first formation to limit the depth of insertion of the neck into the bore by a different amount, characterised in that the plurality of second formations are angularly disposed around the longitudinal axis and the first formation and plurality of second formations slidingly engage when the first trial head is removed or detached from the stem component by translating along the longitudinal axis.

Hence, the offset of an assembly of a trial head of a particular size and the stem component can be varied during trialling. Also, the mechanism by which the offset is adjustable allows the trial head easily to be removed from the stem component and so other trial heads can also be used with the same stem component having different offsets and/or different sizes thereby allowing a range of trialling sizes and offsets to be provided to cover much or all of the typical range of patient sizes but with a reduced number of trialling parts.

Preferably, the assembly can be adjusted without fully separating the trial head and stem. The first formation and plurality of second formations can be positioned on the neck and within the bore so as to be slidingly disengagable while an end portion of the free end of the neck is still located within the bore of the first trial head. The trial head can be rotatable about the free end of the neck while the free end of the neck is within the bore and with the first formation disengaged from the plurality of second formations

The first formation or plurality of second formations can terminate short of an end most face of the free end of the neck or a mouth of the bore of the trial head.

The first formation and plurality of second formations can terminate short of an end most face of the free end of the neck and a mouth of the bore of the trial head.

The first formation can terminate short of an end most face of the free end of the neck or a mouth of the bore of the trial head.

The first formation can comprise a lug and/or the second formations can comprise slots.

The first formation/pair of formations can be provided on the free end of the neck. The plurality of second formations/pairs of formations can be provided within the bore of the first trial head.

The first formation/pair of formations can be within the bore of the first trial head. The plurality of second formations/pairs of formations can be provided on the free end of the neck.

The first formation/pair of formations can comprise male formations and the second formations/pairs of formations can comprise female formations. The first pair of formations can comprise a pair of lugs or splines. Each of the second pairs of formations can comprises a pair of slots. Each pair of slots can have a different length.

Having the male formation or formations on the trial head can be preferred. This can help to prevent the trial head from being used with the prosthetic stem and so would prevent trialling off the prosthetic stem.

The first formation/pair of formations can comprise female formations and the second formations/pairs of formations can comprise male formations. The first pair of formations can comprise a pair of slots. Each of the second pairs of formations can comprise a pair of lugs or splines. Each pair of lugs or splines can have a different length and/or a different position along the longitudinal axis.

The trial implant system can include a plurality of trial heads. The plurality of trial heads can include heads of the same size but with differently configured first formations or pairs of formations and/or second formations or pairs of formations so that each head of the same size can provide a different set of adjustable offsets. The plurality of trial heads can include heads of different sizes and with first formations/pairs and/or second formations/pairs of formations providing the same set of adjustable offsets as the first trial head or a different set of adjustable offsets.

The trial implant system can further comprise a second trial head having an articulating surface. The second trial head can include a bore having a bore longitudinal axis and which can receive the free end of the neck with the neck longitudinal axis and bore longitudinal axis coincidental or aligned. The second trial head can be a different size to the first trial head. The second trial head can includes either a further first formation/pair of formations or a further plurality of second formations/pairs of formations angularly disposed around the longitudinal axis.

The trial implant system can further comprise a second trial head having an articulating surface. The second trial head can include a bore having a bore longitudinal axis and which can receive the free end of the neck with the neck longitudinal axis and bore longitudinal axis coincidental or aligned. The second trial head can be the same size as the first trial head. The second trial head can includes either a further first formation/pair of formations or a further plurality of second formations/pairs of formations angularly disposed around the longitudinal axis. The further first formation/pair of formations or the further second formations/pairs of formations can limit the depth of insertion of the neck into the bore by different amounts which differ from those of the first trial head.

The first trial head can include a plurality of markings. Each marking can correspond to a different offset associated with a respective one of the second formations/pairs of formations.

The plurality of markings can be provided on a side part of the articulating surface. The plurality of markings can be angularly disposed about the bore longitudinal axis.

The neck can include a plurality of markings, each marking corresponding to a different offset associated with a respective one of the second formations/pairs of formations. The plurality of markings can be disposed at different positions along the neck longitudinal axis.

The plurality of markings can be arranged to provide a scale. The scale can include graduations. The markings can include an indication of the magnitude of each offset and/or the sense of each offset.

Each second pair of formations can be arranged in diametric opposition around the longitudinal axis.

There can be at least three second formations or second pairs of formations, at least four pairs, more preferably at least five pairs and most preferably at least six pairs.

The stem component can comprises a trial stem or a part of a trial stem.

The stem component can comprise a part of a trial stem and can include an attachment mechanism for releasably attaching the part of the trial stem to a broach or a part of a broach. The system can also include a broach having one or more features of an attachment mechanism arranged to interact with the trial stem to releasably attach the stem component to the broach or a part of a broach.

The stem component can comprise a prosthetic stem or a part of a prosthetic.

The trial implant system can further comprise a protector which is removable attached to the free end of the neck. The first formation/pair of formations or the plurality of second formations/pairs of formations can be part of or on or provided by the protector.

The protector can include a side wall and the first formation/pair of formations can extend from the side wall on opposed sides.

The protector can includes an end wall and which can be arranged to cover the free end of the neck. The end wall can also be arranged to abut against an end face or surface of the free end of the neck.

Embodiments of the invention will now be described in detail, by way of example only, and with reference to the accompanying drawings, in which:
Figures 1A to 1D show various views of a head part of a first embodiment of a trial system according to the invention;
Figures 2A to 2D show various views of a neck part of the trial system of the first embodiment of the invention;
Figures 3A to 3C show perspective views of the trial system of the first embodiment of the invention in use;
Figures 4A to 4C shows various views of the trial system of the first embodiment of the invention adjusted to provide different amounts of offset;
Figures 5A to 5D show various views of a head part of a second embodiment of a trial system according to the invention;
Figures 6A to 6D show various views of a neck part of the trial system of the second embodiment of the invention;
Figure 7 shows a side view of the trial system of the invention illustrating marking on the head component;
Figure 8 shows a perspective view of the neck component of the trial system illustrating neck marking;
Figure 9 shows a side view of the trial system using the neck component of Figure 8;
Figure 10 shows a perspective view of parts of an example trial system;
Figure 11 shows a cross sectional view through parts of the example trial system of Figure 10;
Figure 12 shows a cross sectional view through the prosthetic components of the example trial system of Figure 10;
Figure 13 shows a cross sectional view through parts of a second version of the example trial system;
Figure 14 shows a cross sectional view through parts of a third version of the example trial system;
Figure 15 shows a perspective view of parts of a third embodiment of the trial system of the invention;
Figure 16 shows a cross sectional view through parts of the third embodiment of the trial system as shown in Figure 15;
Figure 17 shows a schematic perspective view of a taper protector and neck parts of a fourth embodiment of the trial system of the invention;
Figure 18 shows a schematic perspective view of a taper protector and neck parts of a fifth embodiment of the trial system of the invention;
Figure 19 shows a schematic perspective view of a taper protector and neck parts of a sixth embodiment of the trial system of the invention;
Figure 20 shows a schematic perspective view of a seventh embodiment of a protector part of the trailing system of the invention;
Figures 21A to 21D show various views of a head part, neck part and trial system according to an eighth embodiment of the invention; and
Figures 22A to 22E show various views of a head part, neck part and trial system according to a ninth embodiment of the invention.

Some embodiments of the invention will now be described by way of examples to provide an overall understanding of the principles of the structure, operation and use of the devices and methods disclosed herein. One or more examples of these embodiments are illustrated in the accompanying drawings. Those of ordinary skill in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments.

Although the invention will be described below with reference to a hip implant, it will be appreciated that the invention can also be applied to other ball and socket type joint implants, such as a shoulder implant. In the following, "prosthesis" or "prosthetic" will be used to denote the actual implant finally placed by the surgeon to replace the patient's joint and "trial" will be used to denote for implants temporarily placed or used by the surgeon during the procedure for trialling purposes.

A first aspect of the invention aims to reduce the number of trial head components required to enable trialling of the different offsets and/or head sizes and/or taper sizes available. This is achieved by adapting the neck and trial head to allow the trial head to be attached at different depths to the neck and which correspond to the different offsets available of that head size. For example, for existing implant systems many different trial heads may be needed, for example up to twenty one to provide the different combinations of offset, taper size and head size needed to allow reliable trialling. However, this could be reduced to merely 7 different trial heads; one 28mm trial head; two 36mm trial heads; two 40mm trial heads; and two 44mm trial heads, with each trial head offering three different offsets. Fewer than seven different trial heads could be provided if more than three offsets are provided by the trial heads, particularly by the larger trial heads.

A second aspect aims to improve the reliability of trialling of ball and socket type joints, by allowing the surgeon to decide to use the prosthetic stem implant component for trialling rather than a trial stem component. A stem taper protector and specially adapted trial head component are used in which the trial head is configured to interact with the stem taper protector so that when assembled the prosthetic stem and trial head assembly has the same geometry as the prosthetic stem and a prosthetic head corresponding to the trial head. The stem taper protector allows the prosthetic stem to be used for trialling while protecting the stem taper from damage and/or being exposed to fluids from the surgical site which might otherwise decrease the reliability of the mating between the prosthesis stem taper and the prosthesis head component.

The first and second aspects can also be combined to provide offset adjustment and stem taper protection.

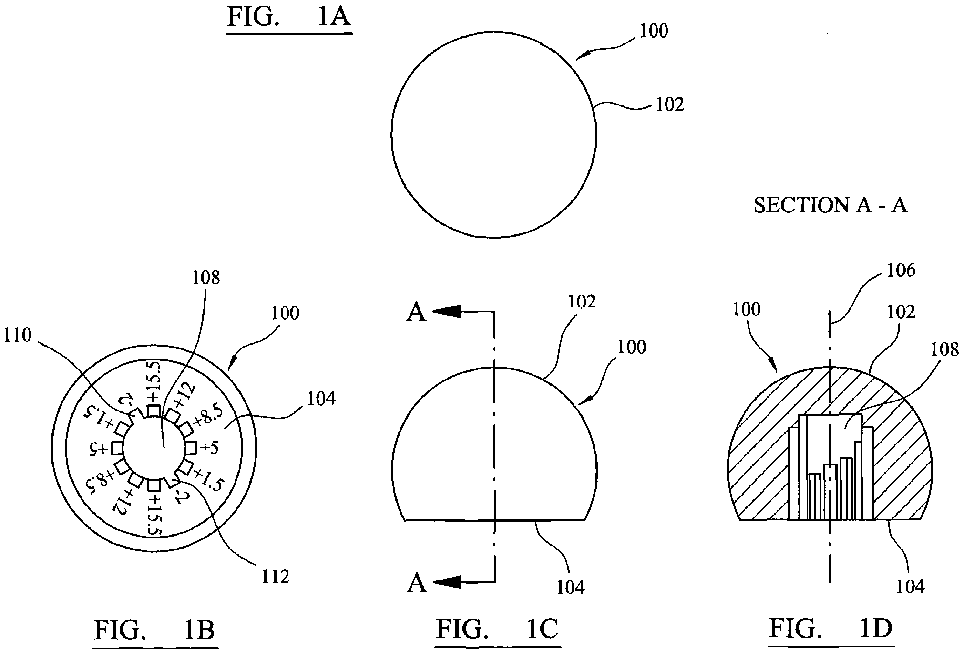

With reference to Figures 1A to 1D there are shown respective plan, bottom, side and cross sectional views of a first embodiment of a trial head 100 being a part of a trial implant system according to the first aspect of the invention. Trial head 100 has the form of a truncated sphere and has an articulating surface 102 extending over side and upper parts of the surface of a sphere. A lower surface 104 has a flat generally circular form. The trial head 100 has a longitudinal axis 106 passing through the centre of the lower circular surface 104 and through a pole in the upper part of the articulating surface 102 about which the articulating surface is generally rotationally symmetric.

As best illustrated in Figures 1B and cross section 1D (along line A-A of Figure 1C), a right circular cylindrical bore 108 is defined by an inner wall within the trial head and extends along the longitudinal axis. The inner wall of the trial head includes twelve slots, extending along the longitudinal axis 106 and arranged equi-angulalry around the longitudinal axis. The slots of each pair of the six opposed pairs of slots, e.g., slots 110 and 112, have the same length, and each pair of the six pairs of slots has a different length as best illustrated in Figure 1D. Each slot of a pair of slots is arranged diametrically opposite each other about the bore.

The trial head can be made of any suitable material, such as metals, alloys or plastics, particularly polymers. Preferably, the trial head is made from a material unlikely to damage the articulating surface of a socket component, in case a user trialled against a prosthetic implant, such as an acetabular cup. For example, the trial head can be made from a glass filed polyaryletherketone (PAEK) such as that provided under the name AvaSpire (a trade mark which may be registered in some countries), polyetheretherketone (PEEK) or polyphenylsulfone (PPSU) such as that provided under the name Radel (a trade mark which may be registered in some countries). The trial head 100 is provided in a plurality of different sizes, with each size corresponding to the size of a corresponding prosthetic head. The size of the head can be defined by the diameter of the spherical part. For example, the trial and prosthetic heads can be provided in the sizes 28mm, 36mm, 40mm and 44mm.

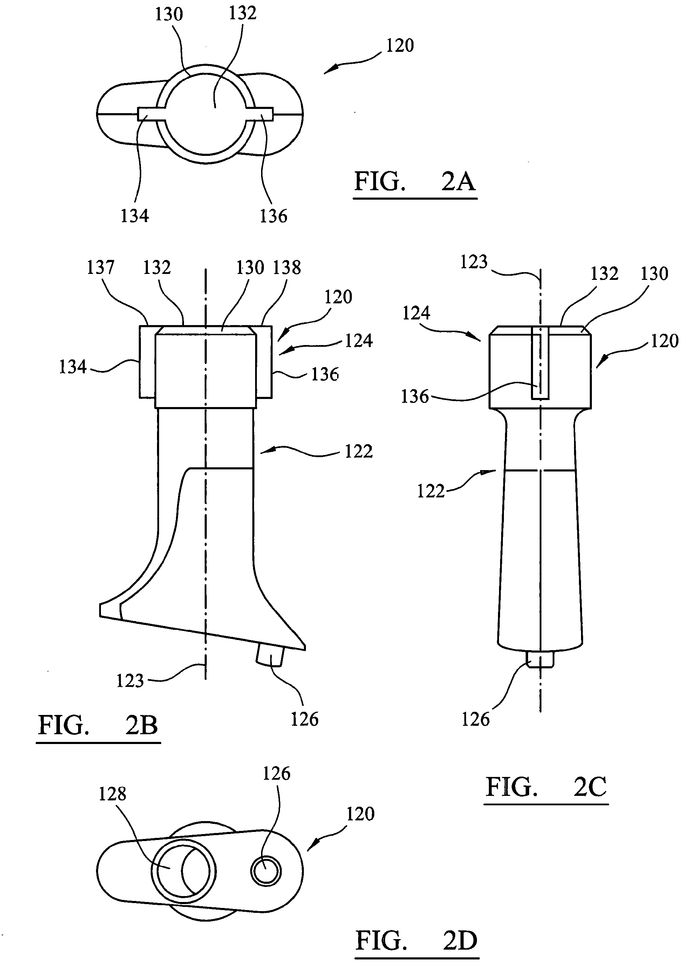

Figures 2A to 2D show respective plan, side, end and bottom views of a first embodiment of a stem component 120 being a part of the trial implant system according to the first aspect of the invention. The stem component includes a neck 122 having a free end 124 and a flared base 126. An underside of base 126 includes a first male attachment formation 126 and a second female attachment formation 128 for releasably attaching the stem component to mating features of a broach which has been used to prepare a cavity in a long bone to receive the stem prosthesis. The free end 124 of neck 122 has a generally right circular cylindrical form with a chamfered upper edge 130 extending around a top most circular surface 132. Neck 122 has a longitudinal axis 123 passing along its length and through the centre of circular surface 132. A first lug 134 and a second lug 136 extend from the side wall of the free end and provide a pair of lugs arranged diametrically opposite each other. The lugs have the same thickness and their thickness is similar to the width of each of the slots in the trial head so that the pair of lugs can be snuggly and slidingly received within each of the pairs of slots.

In an alternate embodiment, the stem component 120 is provided as part of a stem trial implant rather than being a separate part which is attachable to a broach.

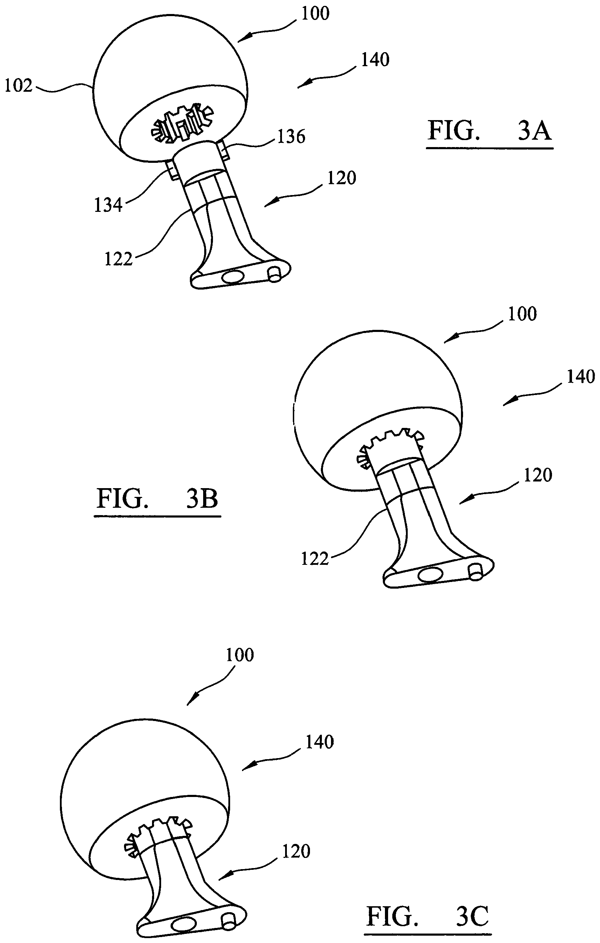

Figures 3A to 3C show respective perspective views of an assembly 140 of the trial head 100 and the stem component 120 during different stages of use. As illustrated in Figure 3A, the longitudinal axis 106 of bore 108 is aligned with the longitudinal axis 123 of neck 122 and with the trial head 100 rotated so as to align a one of the pairs of slots with the pair of lugs 134, 136 of the neck. The neck 120 is then introduced into bore 108 with the pair of lugs being slidingly received within respective slots of the pair of slots. The neck is progressed into the bore until upper ends 137, 138 of the lugs abut respective closed ends of the pair of slots to prevent further translation of the neck within the bore as illustrated in Figure 3C. This then controls the depth of insertion of the neck into the trial head and therefore the offset of the assembly of trial head and stem component, with the trial head at a particular angular position. The offset for the assembly is generally the difference between the extent of the implant assembly in a medial-lateral direction compared to a reference extent (a 'zero offset', although the implant assembly still has a finite extension in the medial lateral direction).

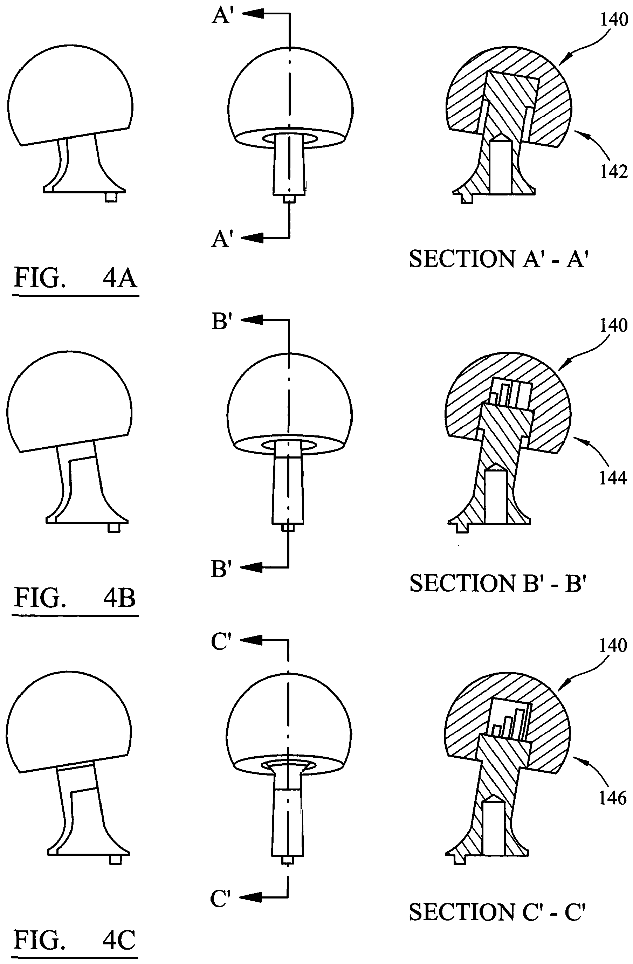

Figures 4A to 4C each show respective side, end and cross sectional views of assembly 140 with different amounts of offset. As each pair of slots has a different length, by changing the angle of rotation of the trial head about the longitudinal axis, a different one of the pairs of slots can be selected to be engaged by the pair of lugs to control the depth of insertion of neck 122 into bore 108. Figure 4A shows assembly 140 in a first maximal offset configuration 142 with the trial head in a first angular position in which the pair of lugs 134, 136 are received within a longest pair of slots of the six pairs of slots and corresponding to an increase in offset of 15.5mm. Figure 4B shows assembly 140 in an intermediate offset configuration 144 with the trial head in a second angular position in which the pair of lugs 134, 136 are received within a different pair of slots of the six pairs of slots and corresponding to an increase in offset of 5mm. Figure 4C shows assembly 140 in a minimal offset configuration 146 with the trial head in a third angular position in which the pair of lugs 134, 136 are received within a shortest pair of slots of the six pairs of slots and corresponding to a reduction in offset of 2mm.

As best illustrated in Figure 1B, the trial head includes six pairs of slots of different lengths and hence six different offsets (-2mm, + 1.5mm, +5mm, +8.5mm, +12mm and +15.5mm) can be selected by changing the angular position of the trial head to one of six discrete angular positions. It will be appreciated, that in order to change the offset of the assembly, the trial head is translated away from the stem a sufficient distance to fully disengage the lugs from open ends of the slots. The trial head can then be rotated about its longitudinal axis to select a different offset and present a different pair of slots to the lugs. The trial head is then translated toward the stem component, with their respective longitudinal axes aligned, so as to engage the lugs with the different pair of slots and hence configure the assembly with an offset different to that of the previous configuration of the assembly.

As also illustrated in Figure 1B, the assembly can include markings showing the amount of offset associated with each of the different pairs of slots. In the first embodiment, the markings are provided on the under surface 104 of the trial head adjacent the open ends of the slots corresponding to the offsets. As shown in Figure 1B the markings can be numerical to indicated the amount of the offset and can also include a sign (positive or negative) to indicated whether the offset is an increase relative to an arbitrary zero (positive) or a decrease increase relative to the arbitrary zero (negative). Hence, for example, the pair of slots 110, 112 correspond to a reduction in offset of 2mm.

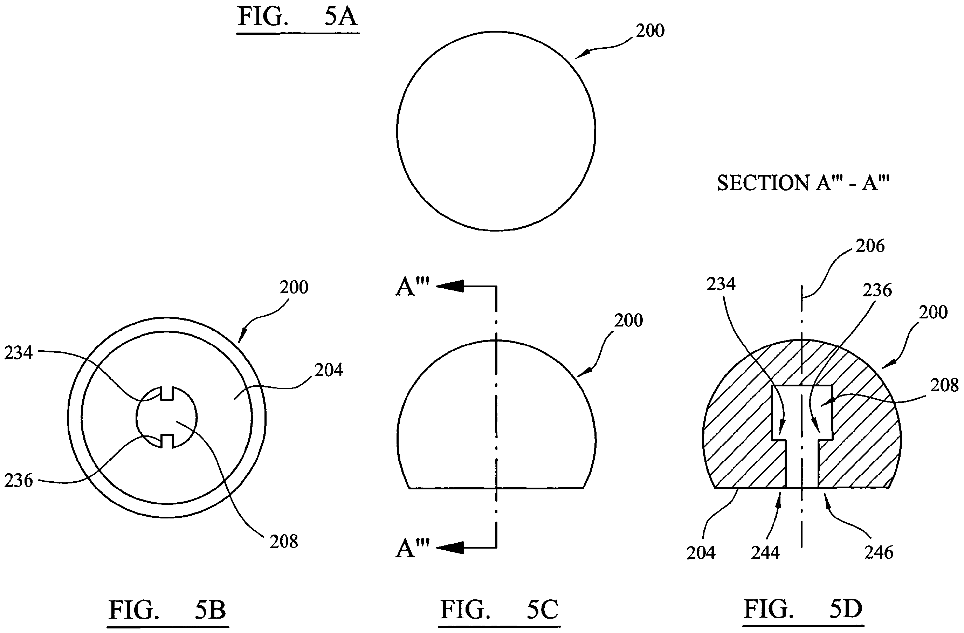

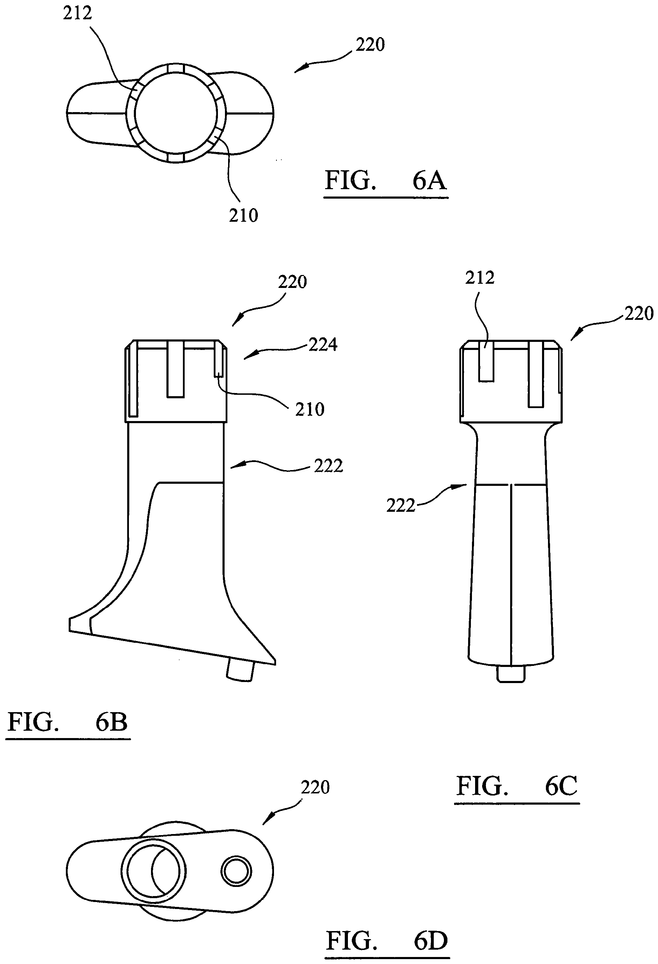

With reference to Figures 5A to 5D there are shown respective plan, bottom, side and cross sectional views of a second embodiment of a trial head 200 being a part of a trial implant system according to the first aspect of the invention. Also, Figures 6A to 6D show respective plan, side, end and bottom views of a second embodiment of a stem component 220 being a part of the trial implant system according to the first aspect of the invention. The second embodiment of the trial head 200 and the stem component 220 are similar in construction to the first embodiment described above. However, in the second embodiment, the pair of lugs 234, 236 are provided on the trial head 200 and the plurality of pairs of slots, e.g. pair of slots 210, 212, are provided on the stem component 220.

As best illustrated in Figure 5D which is a cross sectional view along line A"' - A"' of Figure 5C, a pair of lugs 234, 236 extends from opposed sides of an inner side wall of the trial head 220 which defines an otherwise generally circular cylindrical bore 208 extending along a longitudinal axis 226 of the trial head 200. As illustrated in Figure 5D, the lugs extend from a lower edge 244, 246 flush with lower surface 204 to part way along bore 208. As illustrated in Figures 6A, 6B and 6C, the free end 224 bears 6 slots in total and which provide three pairs of slots, each pair having a different slot length thereby controlling the depth of insertion of neck 222 into bore 208 by abutment of lug lower ends 244, 246 with the closed ends of a one of the pairs of slots, e.g. slots 210, 212.

The position of lower edges 244, 246 and/or the length of the lugs along the longitudinal axis 206 can be different. The position of the lower edges 244, 246 simply acts together with the position of the closed ends of the slots to control the depth of insertion of the neck 222 into bore 208.

It will be appreciated that the trial head 100, 200 has the same size as the corresponding prosthetic head that it is being used to trial for and hence presents the same articulating surface as the prosthetic head. Also the positions and lengths of the slots and lugs are arranged such that the trial assembly when configured to have a selected one of the offsets, e.g. +15mm, will have the same geometry and present the articulating surface at the same position and orientation as the prosthetic stem and head assembly with the corresponding offset, in this example +15mm.

In the first embodiment, markings indicating the offset associated with a particular one of the pairs of slots are on the lower surface 104 of the trial head. However, in other embodiments other marking positions can be used, for example to increase the ease with which the markings can be seen by a surgeon while using the trial assembly.

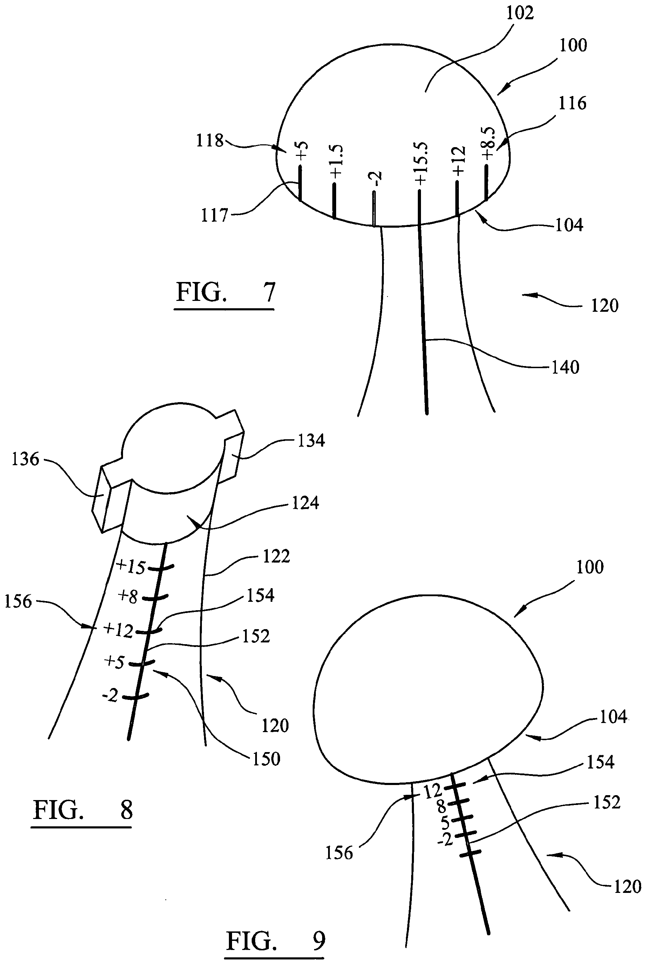

Figure 7 illustrates an alternative, or additional, marking scheme which can be used with the first embodiment. The head 100 includes a plurality of markings 116 on a lateral or side portion of the articulating surface 102 and adjacent a junction with the lower surface 104. The plurality of markings 116 provide a scale from which an offset corresponding to a currently selected one of the pairs of slots can be read off. The markings 116 include a dash 117 and an indication of the sense (increase or decrease) and magnitude 118 of the offset placed to coincide with the position of the corresponding slot and arranged to abut the longitudinal axis 106 of the trial head. Equivalent markings are provided on the opposite side of the head and at diametrically opposed positions. The neck 120 includes a single line marking 140 extending along the longitudinal axis 123 of the neck and aligned with a plane passing through the pair of lugs 134, 136. Hence, the marking 140 provides a reference or datum indicating the angular position of the lugs on the neck and which can be used to indicate the offset associated with a current configuration of the neck 120 and trial head 100 by reading off the one of the head markings 116 aligned with reference line 140, which is +15.5mm in the example illustrated in Figure 7.

Figures 8 and 9 illustrate a further alternative, or additional, marking scheme which can be used with the first or second embodiment of the trial assembly. As illustrated in Figure 8, neck 124 of stem 120 includes a plurality of markings 150. The plurality of markings 150 provide a scale from which an offset corresponding to a currently selected one of the pairs of slots can be read off. The plurality of markings 150 include a linear marking 152 extending along the longitudinal axis 123 of the neck and a plurality of gradations, e.g. graduation 154, and an indication of the sense (i.e. increase or decrease in offset) and magnitude, e.g. 156, associated with each graduation. As illustrated in Figure 9, the graduations and markings are spaced along line 152 such that when the lugs 136, 134 are mated with and abutting the end of one of the pairs of slots, the position along the longitudinal axis of the lower face 104 of the trial head 100 is adjacent to the graduation 154 and indication 156 of the offset corresponding to the currently selected pair of slots, which in the illustrated example is +12. Hence any markings on the scale above are occluded by the trial head, while markings on the scale below are visible. Hence, the underside of the head and junction with the lateral part of the articulating surface act as the reference or datum by which to read from the scale the 'last' visible marking and which is adjacent the underside of the trial head.

Use of the trial implant system in the context of a hip arthroplasty procedure will now be described. The system can include a plurality of trial heads 100 of different sizes so as to accommodate the typical range of patient sizes. The system can also include trial heads of the same size but each having a different plurality of slots so that a full range of offsets is available for any particular head size. Although the trial implant system can include a plurality of trial heads, during use of the system not all of the trial heads may be used and indeed only a one of the trial heads may be used.

The femoral head is resected and a broach or sequence of broaches is used to create a cavity in the superior part of the femur. The broach includes a broach part and a releasably attachable handle. Once the cavity has been formed in the femur to the appropriate size and depth for the planned stem prosthesis, the broach part is left in the cavity and the broach handle is removed. The trial stem component 200 is then attached to the broach using attachment formations 128 and 126 which mate with interacting features on the broach. If the acetabulum is also being replaced, then the acetabulum is prepared and typically a trial cup is placed in the prepared acetabulum. The surgeon the selects a trial 100 head having the same size, i.e. diameter, as the trial cup and attaches the trial head to the stem component 100 at a first offset by engaging lugs 134, 136 with a first pair of slots. The surgeon may then reduce the joint to see whether the first offset and/or trial head size is appropriate. If the first offset is not appropriate for whatever reason, then the surgeon can remove the trial head, rotate the trial head and re-attach the trial head to select a different offset. The surgeon can then reduce the joint again to see if the newly selected offset is more appropriate. If the surgeon wants to try an offset not supported by the current trial head, then the surgeon can select another trial head with the same size but providing a different plurality of offsets. In that case, the surgeon removes and discards the first trial head, selects the new trial head, of the same size, and attaches the new trial head using the pair of slots corresponding to the offset to be trialled. Additionally, or alternatively, the surgeon may determine that the trial head size is not appropriate and may replace the initial trial cup with a further trial cup having a different size, for example larger, and then select a trial head suitable for use with the further trial cup. The surgeon may vary the combinations of offset and head/cup size until they are satisfied with the selected components and may then replace the trial components with prosthetic components having the determined size and offset.
Hence, the first aspect invention reduces the complexity and/or number of trial implants that are supplied while still allowing reliable trialling over a full range of typical patient sizes by providing a simple offset adjustment mechanism which can also allow the number of trial heads supplied into the operating room to be reduced.

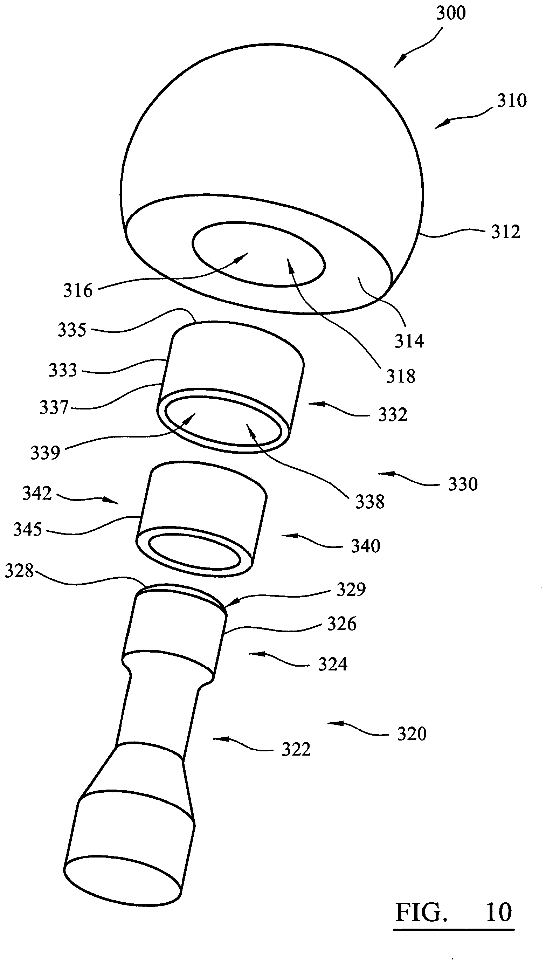

A second aspect will now be described with reference to Figures 10 to 20, in particular. The second aspect also relates to trialling of ball and socket type joints. In particular the second aspect provides the surgeon with the option to decide to use the prosthetic stem component, rather than a trial stem component, for trialling with one or more trial head components.

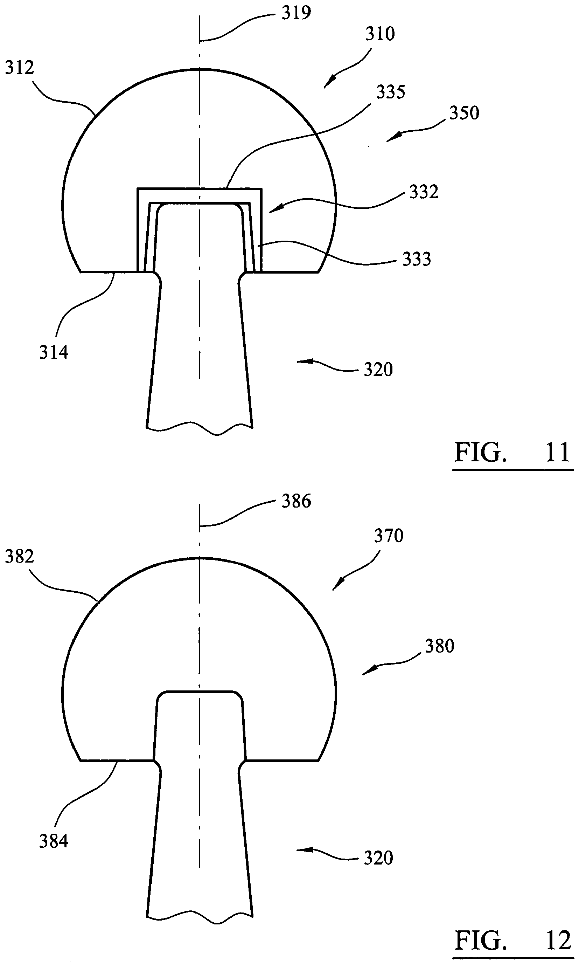

Figure 10 shows an exploded perspective view of a trial head part 310, prosthetic stem part 320 and stem protector part 330 of a trialling system 300 according to the second aspect of the disclosure. Figure 11 shows a cross sectional side view through a trial assembly 350 of the trialling system. Figure 12 shows a cross sectional view through a prosthetic assembly 370 of the trialling system including the prosthetic stem part 320 and a prosthetic head 380.
The trial head 310 generally has the form of a truncated sphere and presents an articulating surface 312 extending over upper and side portions of the trial head. The trial head also includes a planar circular under surface 314. An inner wall 316 defines a circular cylindrical bore 318 which extends into the interior of the trial head along a longitudinal axis 319 which passes through the centre of under surface 314 and the pole at the top of articulating surface 312. The trial head has the same size as the prosthetic head as its spherical part has the same diameter as the spherical part of the prosthetic head 380. The trial head can be made from any suitable biocompatible material including plastics or polymers.
Figures 10, 11 and 12 show only the neck part of the prosthetic stem component 320 for the sake of clarity, although it will be appreciated that in practice the prosthetic stem also includes a main body part and shaft which extends into a cavity formed in the superior part of the femur. The neck part 322 of the prosthetic stem 320 includes a taper 324 toward a free end of the neck. The taper has a smooth side wall 326, and a circular end wall 328 and a chamfer 329 extending around the junction thereof. The finish of the taper side wall 326 is smooth so as to provide a strong interference fit with a corresponding tapered bore in the prosthetic head 380. The prosthetic stem can be made from a suitable biocompatible material such as a metal, an alloy or a plastic. For example, the stem can be made from forged or wrought titanium alloy.

The taper protector 330 has a first outer part 332 and a second inner part 340. Outer part 332 is made of a non-deformable rigid material. Outer part 332 has the form of a generally circular cylindrical cap with a side wall 333 and a circular end wall 335. An outer surface 337 of the side wall 333 is cylindrical and an inner surface 338 is slightly inclined and defines a tapered cavity 339 within the outer part 332. The outer part can be made from a suitable rigid or non-deformable biocompatible material such as a plastic or a metal or alloy. Various different engineering plastics can be used. Suitable plastics include silicone (especially with a high shore hardness), polyphenylsulfone (PPSU), such as that provided under the trade mark RADEL (which may be registered in some countries), Acetal and PEEK (Polyether Ether Ketone). Suitable metals and alloys include stainless steel, titanium and cobalt chromium alloys.

The inner part 340 is made from a deformable material and in particular can be made from a resiliently deformable material. The material of the inner part 340 is can be non-shedding and preferably will leave no-contamination or residue on the taper surfaces 326, 328. The inner part has the form of a generally tapered sleeve or tube, with open ends, and includes a tapering side wall 342. An outer surface 345 of the side wall 342 interfaces with the inner surface 338 of the outer part 332. An inner surface 346 of the side wall 342 interfaces with the outer surface 326 of the taper 324. The inner part 340 can be made from various deformable materials, such as silicone elastomer (with a low shore hardness), urethane, synthetic rubber, such as polyurethane runner, and various foams. The inner part 340 has a lesser shore hardness than the outer part 330.

As best illustrated in Figure 11, the inner part 340 is attached within the outer part 330 and secured therein mechanically and/or by bonding, for example by an adhesive. The surfaces between the inner and outer part may be roughened to provide a better surface for an adhesive to key to. The inner part 340 and outer part 330 may be both made of the same type of material, e.g. silicone, but with different shore hardness, and they may be joined by a silicone adhesive so that the protector has a generally unitary construction.

As also well illustrated in Figure 11, the protector provides a deformable cavity or bore into which the taper 324 of the neck 322 is received and provides an interference fit by which the protector 330 is releasably secured to the taper 324. The protector then presents a non-deformable or rigid outer surface which can then be mated within the bore 318 of the trial head so as to protect the taper surfaces 326, 328 from being exposed to bodily fluids and/or potentially damaging contact with instrumentation or implants during trialling using the prosthetic stem 320.

With reference to Figure 12 there is shown a prosthetic assembly 370 comprising the prosthetic stem 320 and the prosthetic head 380. The prosthetic assembly 370 is assembled after trialling using the trial head 310 and prosthetic stem 320. The prosthetic head 380 has a similar construction to the trial head 310 in that it has a truncated spherical form including an articulating surface 382 and a flat under surface 384. A longitudinal axis 386 passes through the centre of the circular under surface 384 and through a pole toward the top of the articulating surface 382. An inner wall of the prosthetic head 380 defines a tapered cavity or bore which is dimensioned and shaped to provide a taper fit with the taper 324 of the prosthetic stem 320. The prosthetic head 370 can be made from any biocompatible material particularly including metals and alloys. Particularly suitable materials include ceramics, stainless steel, and Cobalt Chrome alloys.

The articulating surface 382 of the prosthetic head 370 is identical to the articulating surface 312 of the trial head 310. A difference between the trial head 310 and prosthetic head 380 is that the cavity 318 in the trial head is adapted to compensate for the presence of the protector 330 on the taper while ensuring that the position of the trial articulating surface 312 relative to the prosthetic stem component 320 is identical to the position of the prosthetic articulating surface 382 to the prosthetic stem 320 in the prosthetic assembly. That is, is Figure 12 and Figure 11 were overlaid, the respective articulating surface 312, 382 would be coincident over the entirety of the spherical surface portion of the trial head 310 and prosthetic head 380.

The structural differences between the trial cavity 318 and prosthetic cavity are that the prosthetic cavity is tapered whereas the trial bore 318 is in the form of a right circular cylinder. Also, the trial bore 318 has a greater lateral dimension and a greater depth so as to accommodate the side walls and end wall of the protector 330. The trial articulating surface 312 should include at least all of the prosthetic articulating surface 382. The trial articulating surface 312 can be greater than the prosthetic articulating surface 382 but should not be lesser so as to ensure that a full range of motion corresponding to that available with the prosthetic component can be trialled. The trial and prosthetic articulating surfaces are sufficiently similar such that the trial head does not behave differently to the prosthetic head.

Hence, as best illustrated by comparing Figures 11 and 12, the trial bore 318 is adapted to accommodate the protector 330 while ensuring that the articulating surface 312 of the trial assembly 350 is at the same position in space as the articulating surface 382 of the prosthetic assembly 370 over preferably the entirety of the common articulating surfaces. In particular, the trial cavity 318 has a greater depth and greater diameter than the prosthetic cavity. Also, the prosthetic cavity is tapered to ensure a good tapered interference fit between the taper 324 and prosthetic head 380 so that the prosthetic head 380 is securely attached to the prosthetic stem 320. The trial cavity 318 is cylindrical such that the trial head 310 can easily be removed from the prosthetic stem and replaced with a different trial head of a different size during the trialling procedure and without removing the protector 330 from the taper 324. The deformable part 340 helps to provide an interference fit between the protector 330 and taper which helps to prevent the protector being prematurely removed from the taper during trialling and before maturing the prosthetic head. Further, as the deformable part 340 is in the form of a sleeve, the non-deformable end wall 335 of the non-deformable part 332 is situated between the end face of the taper and the end surface of the trial bore 318 which ensures accurate seating of the trial head 310 on the prosthetic stem 320 to ensure that the articulating surfaces of the trial and prosthetic heads are congruent. This is preferred compared to having a deformable part between the end surface of the taper 328 and the end face of the trial cavity which could lead to variation in position of the articulating surface of the trial head 310 when not seating on a rigid part.

Furthermore, the rigid outer walls 333 of the outer part 332 helps to retain the deformable part 340 within the protector 330 when the stem taper is initially introduced into the protector and enhances the interference fit by allowing the deformable part to be slightly compressed between the rigid taper surface 326 and the rigid side wall 333 of the outer part.
Hence, in use, the surgeon resects the proximal or superior part of the femur and creates a cavity in the resected femur using a broach or similar instrument. The prosthetic stem can then be placed in the cavity with the protector 330 in place and surrounding the side and end surfaces of the taper 324. The protector 330 can be provided pre-assembled to the prosthetic stem. In that case, the material of the protector is selected such that the protector does not degrade when sterilised or over time. The surgeon can then releasable attach trial heads of different sizes over the protector while being confident that the position of the trial articulating surface 312 will correspond to the position of the ultimately selected prosthetic head. After trialling, and when the surgeon has selected the appropriate head size, the protector 330 is removed from the taper 324 and the prosthetic head 370 is securely fastened to the prosthetic stem component 320 to form the prosthetic assembly 370 as illustrated in Figure 12. Owing to the use of the taper protector 330, the taper is not exposed to bodily fluids which may reduce or otherwise harm the effectiveness of the taper fit between the prosthetic head 370 and prosthetic stem 320. Additionally and / or alternatively, the taper protector 330 may help to avoid any scratches or other damage to the finish of the taper surfaces owing to instrumentation, implants or other equipment used by the surgeon during trialling. Therefore, the reliability of the attachment of the prosthetic head to the prosthetic stem can be improved and any issues associated with contamination or harm to the taper during trialling can be reduced or avoided.

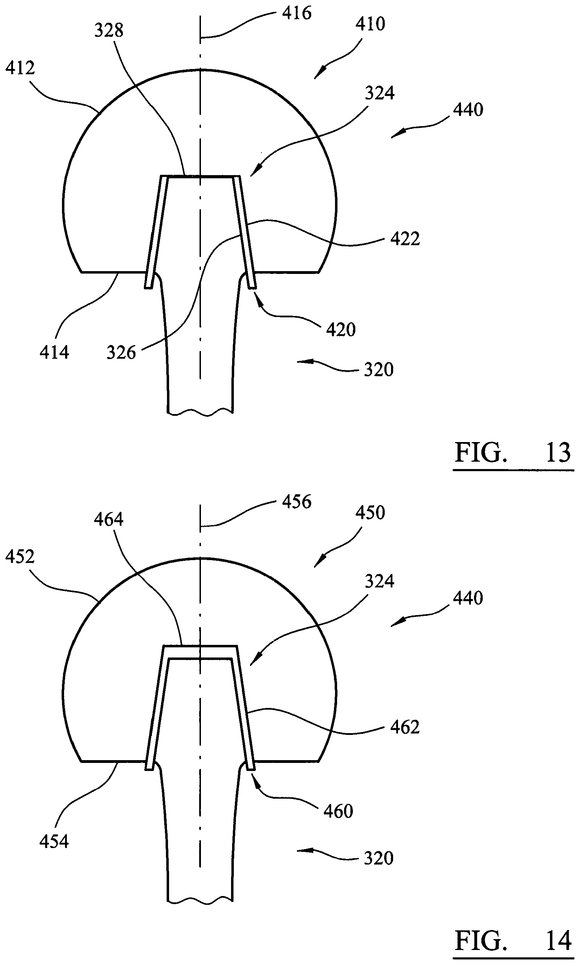

With reference to Figure 13 there is shown a further trial assembly 390. The pile assembly 400 includes prosthetic stem 320 and trial head 410 having a spherical articulating surface 412 and a flat circular under surface 414. Trial head 410 is similar to the trial head 310 but has a tapered trial cavity extending into and along a longitudinal axis 416 of the trial head. Trial assembly 400 includes a protector 420 covering the side wall surface 326 of the taper 324. Taper protector 420 has the general form of a tapering sleeve or tube and comprises an inclined side wall 422. The taper protector 420 has open ends so that the inner end wall of the trial head bore can seat upon the upper end wall 328 of the taper. The taper protector 420 can have a number of constructions. It can be made from a resiliently deformable material or a rigid material or a combination thereof. The taper protector 420 can be made from the same type of material but engineered to have a deformable inner surface and a rigid outer surface. A deformable inner surface helps to provide interference fit between the protector 420 and the taper so as to help retain the protector on the taper during trialling. A rigid outer surface, and the shape of the tapered trial cavity can be engineered to provide a releasable attachment mechanism to allow different trial heads to be attached to and removed from the prosthetic stem 320 without pulling off the taper protector 420. In other embodiments, the sleeve 422 can have a circular cylindrical outer wall and a tapered inner wall similar to taper protector 330 but without the end wall 335. Although schematically illustrated in Figure 13 as being a single piece, in other examples, taper protector 420 may also have a two part construction including a rigid non-deformable outer part and a resiliently deformable inner part, again, similar to stem protector 330.

Figure 14 shows a cross sectional view for a further example of a trial assembly 440. Trial assembly 440 includes prosthetic stem 320 and trial head 350 similar to the previously described trial heads. Trial head 450 has a spherical articulating surface 452 and a flat circular lower surface 454 and a longitudinal axis 456 passing through the centre of circular under surface 454 and a pole of articulating surface 452. Trial head 450 includes inner surfaces defining a tapered trial bore within which a taper protector 460 and taper 324 of the prosthetic stem are received.
Taper protector 460 is similar to the taper protector 420 but has a generally cap-like construction and includes a tapering side wall 462 and a circular end wall 464. Taper protector 460 can be made from a non-deformable material, a deformable material or combinations thereof. Also, taper protector 460 can have a generally unity construction being made from a single part or may be made from two separate parts providing a deformable part and a non-deformable part, similar to taper protector 330. It is preferred if end wall 464 is made largely or entirely of a rigid material so as to provide a well-defined seating of the trial head 450 on the prosthetic stem 320 for the pile assembly 440 for the reasons discussed above.

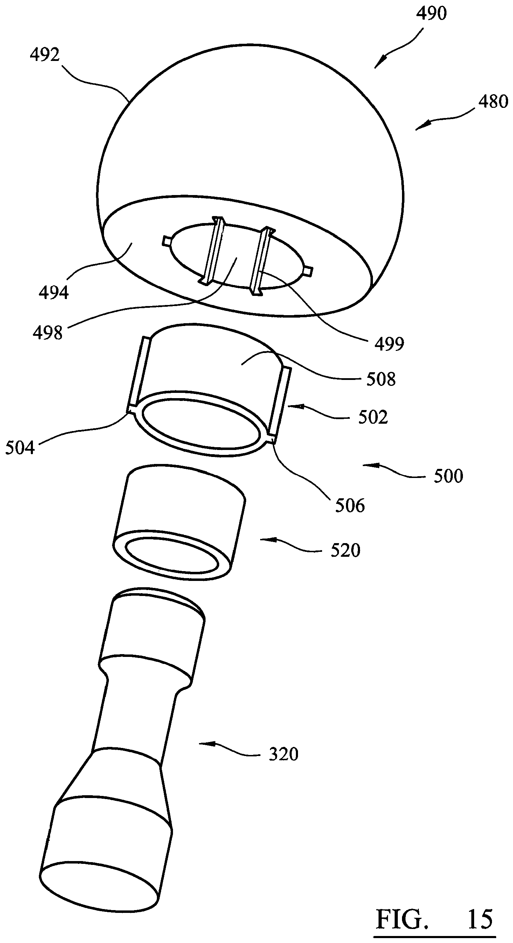

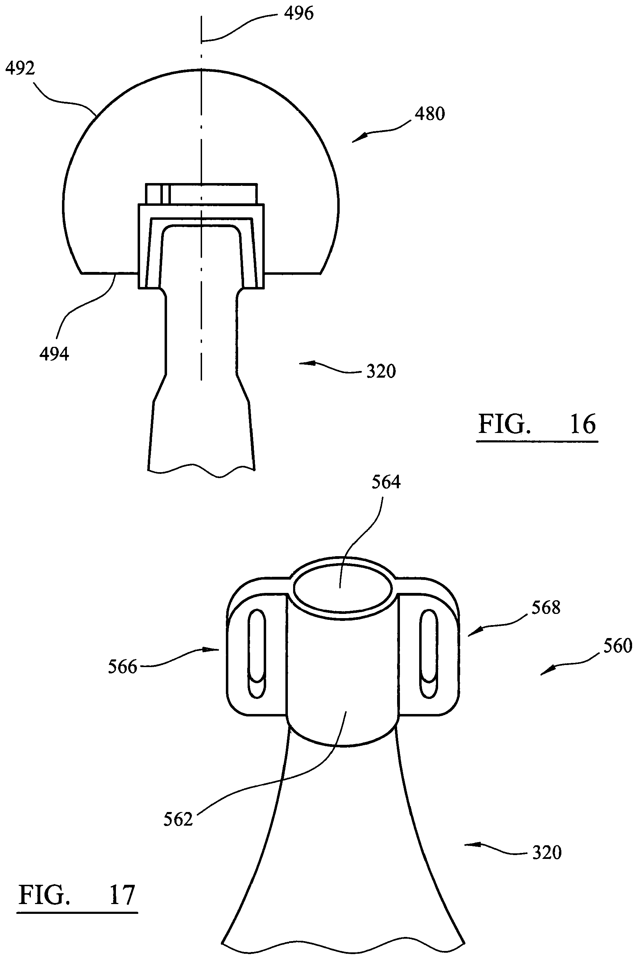

With reference to Figure 15, there is shown an exploded perspective view of a trial assembly 480 combining the first and second aspects of the invention. Figure 16 shows a cross sectional side view of the trial assembly 480. Trial assembly 480 includes a prosthetic stem 320, a trial head 490 and a taper protector 500. Trial head 490 has a spherical articulating surface 492, a circular under surface 494 and a longitudinal axis passing through the centre of circular under surface 494 and a pole of the articulating surface 492. Inner walls of trial head 490 define a trial cavity or bore 498 having a generally circular cylindrical shape. A plurality of slots, e.g. slot 499, are formed in an inner side wall of trial head 490 and define a plurality of pairs of slots of different length similar to those described for the first aspect of the invention.

Taper protector 500 has a similar construction to taper protector 330 and includes a non-deformable outer part 502 and an inner, tapered deformable part 520. Outer part 502 is made from a rigid material and has a cap like construction and also includes a pair or lugs or splines 504, 506 extending from a side wall 508 and diametrically opposed to each other.

Hence, as illustrated in Figure 16, the trial assembly 480 provides the combined benefits of the first and second aspects of the invention of allowing trialling from a prosthetic stem 320 or also providing offset adjustment and the benefits associated therewith described above.

Figure 17 shows a schematic perspective view of a further embodiment of a taper protector 560 mounted on the taper of a prosthetic stem 320. Taper protector 560 is similar to taper protector 500 but includes live spring elements to control the fit between the protector and the trial head. The live springs provide a snug fit between the protector and trial head, while also allowing for variability in the exact sizes owing to tolerances. As illustrated in Figure 17, the taper protector 560 has a generally cap-like form and includes a side wall 562 and a circular end wall 564. Side wall 562 has an outer surface presenting a circular cylindrical form and a tapered inner surface receiving an inner deformable part (not visible in Figure 17). A first 566 and a second 568 lug extends from diametrically opposed sides of the protector 560. Each lug 566, 568 includes an aperture passing therethrough to form the live spring elements.

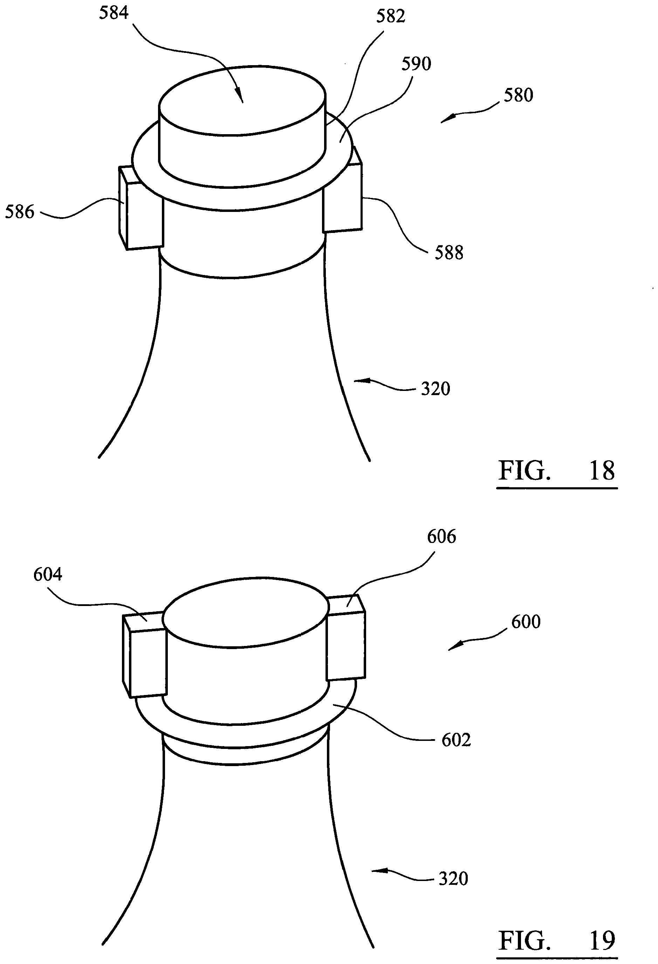

Figure 18 shows a schematic perspective view of a further embodiment of a stem protector 580 mounted on the taper of a prosthetic stem 320. Taper protector 580 has a similar construction to those described previously and has a generally cap-like form including a side wall 582 and a circular end wall 584. Taper protector 580 includes a pair of opposed lugs 586, 588 extending from a lower end of the taper protector 580 toward an intermediate portion and stopping short of the end wall 584. A circlip (sometimes also referred to as a c-clip) 590 is provided between the end wall 584 and an upper end of lugs 586, 588. Alternatively, an O-ring can be used instead of circlip 590. Similarly to the live springs, the circlip or O-ring provide a mechanism for controlling the fit between the protector and the trial head. Put another way, the O-ring or circlip help to control the interference fit between the parts.

Figure 19 shows a perspective view of a yet further embodiment of a taper protector 600 similar to taper protector 580. In the further embodiment, taper protector 600 includes a circlip 602 or O-ring extending around taper protector 600 but below lugs 604, 606. Otherwise, the construction of taper protector 600 is similar to that of taper protector 580 illustrated in Figures 18.

It will be appreciated that the features of protectors 560, 580 & 600 can also be used on the trial stem of the first aspect of the invention.

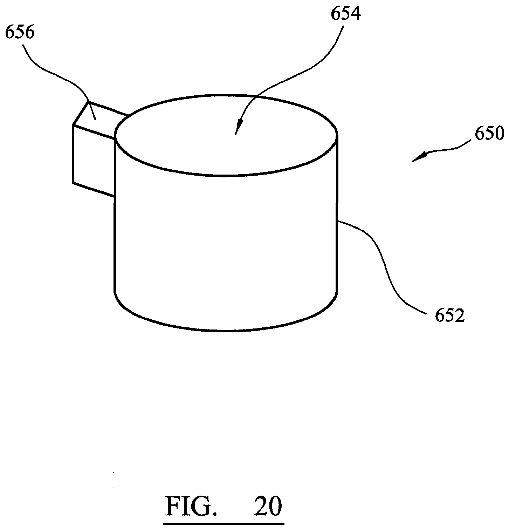

Figure 20 shows a schematic perspective view of a further embodiment of a protector 650. Protector 650 has a generally circular cylindrical cap like construction and includes a side wall 652 and an end wall 654 and a single first formation 656 in the form of a lug extending outwardly form the side wall 656. It will be appreciated that protector 650 also includes a cavity for receiving a taper of a prosthetic stem in an interference fit manner similarly to that described above in connection with the various other embodiments of the protector. However, in this embodiment, protector 650 includes only a single lug rather than a pair of lugs. Hence, the corresponding trial head includes a plurality of individual slots of different lengths, rather than pairs of slots of different lengths. It will be appreciated that a similar arrangement using a single male formation on a trial stem neck can also be used.

Hence, the embodiment illustrated in Figure 20 uses only a single male formation interacting with a plurality of individual female formations, rather than pairs of male and female formations, in order to adjust offset. It will be appreciated that the alternate arrangement of the single male formation being in the cavity of the trial head and the individual female formations being on the protector or stem neck is also possible.

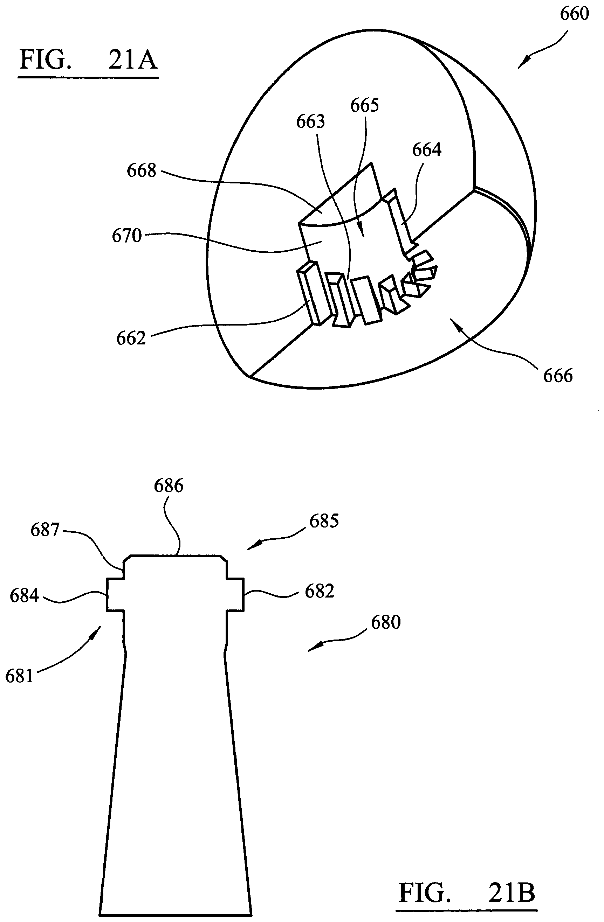

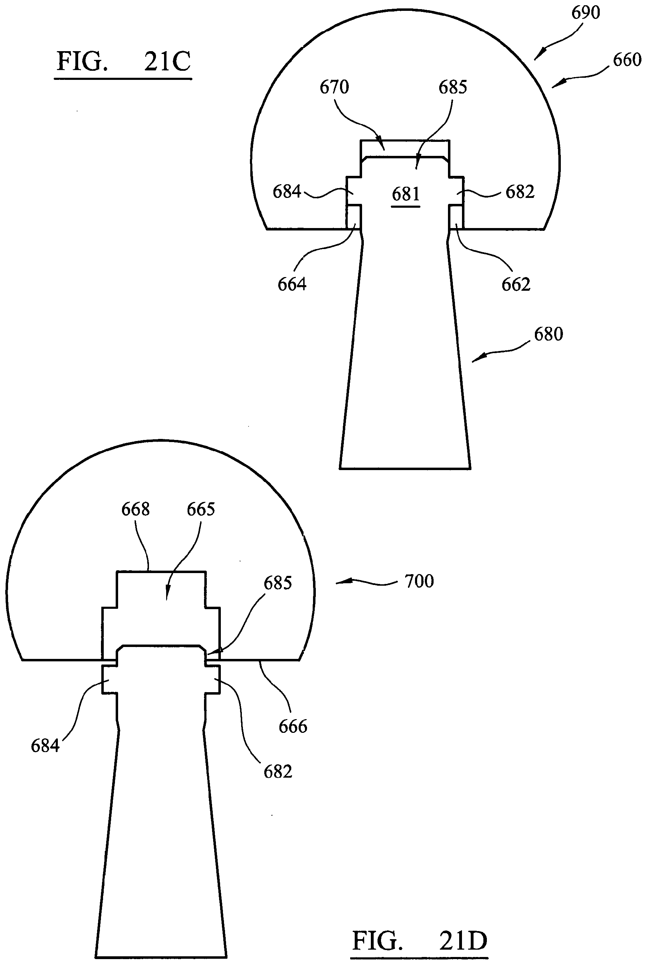

Figures 21A to 21D show various views of a head part, neck part and trial system according to a third embodiment of the invention. Figure 21A shows a perspective view cross sectional view of a trial head 660, Figure 21B shows a side elevation of a stem part 680, Figure 21C shows a side cross sectional view of the trial head and stem part in a fully engaged configuration or state 690 and Figure 21D shows a side cross sectional view of the trial head and stem part in a partially engaged configuration or state 700 in which the free end 685 of the stem 680 is still at least partially located or received within the central bore 665 of the head 660.

The third embodiment is similar to the first embodiment illustrated in Figures 1 to 4 and described above in that the trial head includes seven pairs of slots defined in an inner wall which defines a central, general circular bore 665. Each pair of slots comprises diametrically opposed slots, e.g. slots 662 664, each having the same length. However, each pair of slots has a different length compared to the other pairs of slots. All of the slots extend from an underside or under surface 666 of the trial head in a generally longitudinal direction parallel to the central axis of the trial head and terminate at a closed end which is part way into the central bore but not adjacent the end face 668 which closes the central bore 685. The longest pair of slots, slots 662, 664, extend approximately two thirds of the way into the bore such that approximately a third of the length of the side walls of the bore does not include any slots. This provides an end space 670 for receiving a free end of the stem as described in greater detail below.

The stem 680 is similar to the stem illustrated in Figures 2A to 2D, but the pair of lugs 682, 684 do not extend all the way to the top most surface 686 of the free end 685 of the cylindrical end portion 681 of the stem. Hence, there is a distal most portion of the stem at the free end 685 which does not bear any part of the lugs. The diameter of the cylindrical end portion 681of the stem is slightly less than the inner diameter of the cylindrical bore 665 of the trial head so that the free end 685 can be slidingly received within the end space 670 and also within the remainder of the cylindrical bore 665.

Figure 21C shows a side cross sectional view of the trial head 660 and stem part 680 in a fully engaged configuration or state 690. As can be seen, the lugs 684, 686 are received within a pair of slots 662, 664, and with the cylindrical end portion 681 of the stem located within the cylindrical bore of the trial head and in particular the free end portion 685 accommodated within the end space 670 of the cylindrical bore. The lugs and closed ends of the slots 662, 664 abut to control the depth of insertion of the stem into the trial head and therefore control the length or off set of the assembly. Figure 21C shows the minimal off set arrangement, i.e. using the longest pair of slots 662, 664, and hence the end space 670 has to be sufficiently long to accommodate the length of the free end part 685 of the stem.

In order to adjust the length of the assembly, the trail head is slid along the longitudinal axis, until the lugs 682, 684 have disengaged the slots and are clear of the under surface 666 of the trial head, as illustrated in Figure 21D. However, the free end portion 685 of the stem 685 is still located at least partially within the cylindrical bore 665 of the trial head and the trial head can be rotated about the free end portion 685 as the lands, e.g. land 663, between adjacent slots slide over the sidewall 687 of the free end portion 685. Hence, as illustrated in Figure 21D, the trial head 660 and stem part 680 are in a partially engaged configuration or state 700 in which they are not fully disengaged and in which the trial head can be rotated about the stem so as to allow a different pair of slots to be selected and engaged with the lugs 682, 684 to select a different length and hence off set for the trial assembly.

Allowing the length of the assembly to be changed without fully disengaging the trial head and stem makes it easier for the surgeon to adjust the length of the assembly *in situ* with the joint fully or partially reduced and without having to dislocate and then reduce the joint again. It is preferably to avoid repeatedly dislocating and/or reducing the joint during surgery so as to help avoid damage to the joint, such as to articulating surfaces and/or soft tissues structures, such as tendons and ligaments.

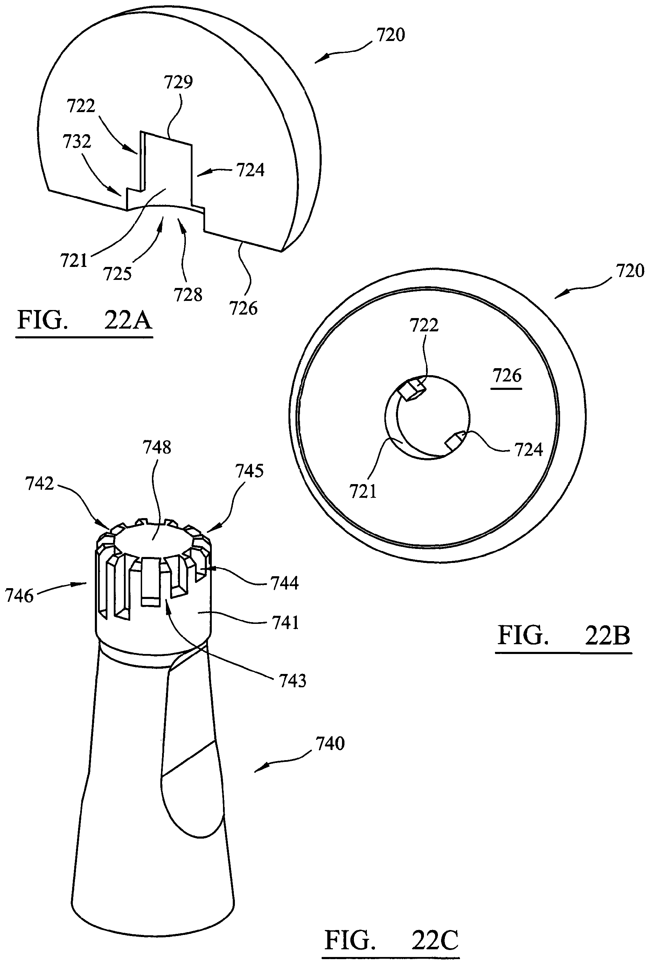

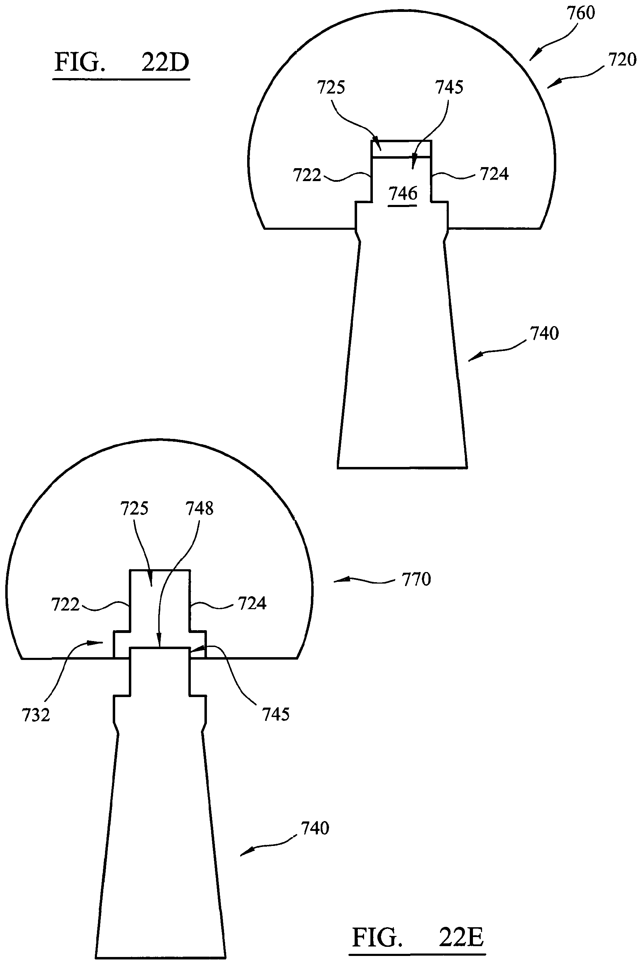

Figures 22A to 22E show various views of a head part, neck part and trial system according to a fourth embodiment of the invention. Figure 22A shows a perspective view cross sectional view of a trial head 720, Figure 22B shows a perspective view of an underside of the trial head 720, Figure 22C shows a perspective view of a stem part 740, Figure 22D shows a side cross sectional view of the trial head and stem part in a fully engaged configuration or state 760 and Figure 22E shows a side cross sectional view of the trial head and stem part in a partially engaged configuration or state 770 in which the free end 745 of the stem 740 is still at least partially located or received within the central bore 725 of the head 720.

With reference to Figure 22A and 22B, the fourth embodiment is similar to the second embodiment illustrated in Figures 5 and 6 and described above in that the trial head 720 includes a pair of lugs 722, 724 extending inwardly from an inner wall 721 which defines a central, general circular bore 725. The trail head 720 is similar to that shown in Figure 5 except the lugs do not extend all the way down to an under surface 726 of the trail head. Rather, the lugs 722, 724 terminate before the under surface 726 at an open end 728 of the bore and not adjacent the end face 726 which defines the mouth to the central bore 725. The lugs extend approximately two thirds to three quarters of the way along the bore from the closed end 729 toward the open end such that approximately a third or a quarter of the length of the side walls of the bore does not include any part of the lugs. This provides an open end space 732 for receiving a free end of the stem as described in greater detail below.

With reference to Figure 22C, the stem 740 is generally similar to the stem illustrated in Figures 6A to 6D, and includes six pairs of slots, e.g. pair of diametrically opposed slots 742, 744, defined in an outer wall 746 which defines a generally circular cylindrical end portion 746 having a free end portion 745. Each pair of slots comprises diametrically opposed slots, e.g. slots 742 744, each having the same length. However, each pair of slots has a different length compared to the other pairs of slots. All of the slots extend from a top most face or surface 748 of the cylindrical end portion 746 of the stem in a generally longitudinal direction parallel to the central axis of the stem and terminate at a closed end which is part way along the end portion 746.

The diameter of the cylindrical end portion 746 of the stem is slightly less than the inner diameter of the cylindrical bore 725 of the trial head so that the free end 745 can be slidingly received within the open end space 732 and also within the remainder of the cylindrical bore 725.

Figure 22D shows a side cross sectional view of the trial head 720 and stem part 740 in a fully engaged configuration or state 760. As can be seen, the lugs 722, 724 are received within a pair of slots and with the cylindrical end portion 746 of the stem located within the cylindrical bore 725. The lugs and closed ends of the slots abut to control the depth of insertion of the stem into the trial head and therefore control the offset of the assembly. Figure 22D shows the minimal off set arrangement, i.e. using the longest pair of slots, and hence the fee end 745 of the stem is received within the bore but separated from the closed end face 729.

In order to adjust the length of the assembly, the trail head is slid along the longitudinal axis, until the lugs 722, 724 have disengaged the slots and are clear of the top end surface 748 of the trial head, as illustrated in Figure 22E. However, the free end portion 745 of the stem 740 is still located at least partially within the cylindrical bore 725 of the trial head, within the open end space 732 and the trial head can be rotated about the free end portion 745 as the lands, e.g. land 743, between adjacent slots slide over the inner wall 721 of the bore. Hence, as illustrated in Figure 22E, the trial head 720 and stem part 740 are in a partially engaged configuration or state 770 in which they are not fully disengaged and in which the trial head can be rotated about the stem so as to allow a different pair of slots to be selected and engaged by the lugs 722, 724 to select a different length and hence off set for the trial assembly.

Hence, this embodiment also allows the length of the assembly to be changed without fully disengaging the trial head 720 and stem 770. The surgeon can therefore adjust the length of the assembly *in situ* with the joint fully or partially reduced and without having to dislocate and then reduce the joint again, thereby reducing the chance of damage to the joint.

It will be appreciated that the same function can also be realised by modifying the slots rather than the lugs. For example the position of the open ends of the slots can be modified so that they terminate before the end most surface of the neck, similarly to the lugs shown in Figure 21B. Various modifications to the positions and/or lengths of the lugs and or slots can be used to allow an end part of the neck to remain in the bore during adjustment of the trial assembly and hence not fully separating the trial assembly during its adjustment.

It will be appreciated that the features of the different embodiments may be modified and/or used in different combinations to those of the embodiments specifically described above.

## Claims

1. A trial implant system comprising:
a stem component (120, 220, 320, 680, 740) having a neck (122, 222) including a free end (124, 224), the neck having a neck longitudinal axis (123);
a first trial head (100, 200, 490, 660, 720) having an articulating surface (102), the first trial head including a bore (108, 208, 498, 725) having a bore longitudinal axis (106, 206) and which can receive the free end of the neck with the neck longitudinal axis and bore longitudinal axis coincident;
a first formation (136, 134, 234, 236, 504, 506, 566, 568, 586, 588, 604, 606, 656, 682, 684, 722, 724); and
a plurality of second formations (110, 112, 210, 212, 499, 662, 664, 742, 744), wherein one of the first formation and the plurality of second formations is provided on the neck and the other is provided within the bore and wherein each of the second formations can matingly engage with the first formation to limit the depth of insertion of the neck into the bore by a different amount, **characterised in that** the plurality of second formations are angularly disposed around the longitudinal axis and the first formation and plurality of second formations slidingly engage when the first trial head is removed or detached from the stem component by translating along the longitudinal axis.

2. The trial implant system of claim 1, wherein the first formation (682, 684, 722, 724) and plurality of second formations (662, 664, 742, 744) are positioned on the neck and within the bore so as to be slidingly disengagable while an end portion of the free end of the neck is still located within the bore of the first trial head.

3. The trial implant system as claimed in claim 2, wherein the first formation (682, 684, 722, 724) or plurality of second formations (662, 664, 742, 744) terminate short of an end most face of the free end of the neck (686) or a mouth of the bore (726) of the trial head.

4. The trial implant system as claimed in any preceding claim, wherein the first formation (136, 134, 234, 236, 504, 506, 566, 568, 586, 588, 604, 606, 682, 684, 722, 724) comprises a first pair of formations and the plurality of second formations (110, 112, 210, 212, 499, 662, 664, 742, 744) comprises a plurality of second pairs of formations.

5. The trial implant system as claimed in any preceding claim, wherein the first formation (134, 136, 504, 506, 566, 568, 586, 588, 604, 606, 656, 684, 682) is provided on the free end of the neck and the plurality of second formations (110, 112, 499, 662, 664) are provided within the bore of the first trial head.

6. The trial implant system as claimed in any of claims 1 to 4, wherein the first formation (244, 246, 722, 724) is within the bore of the first trial head and the plurality of second formations (210, 212, 742, 744) are provided on the free end of the neck.

7. The trial implant system as claimed in claim 4, wherein the first pair of formations comprises a pair of lugs (134, 136, 504, 506, 566, 568, 586, 588, 604, 606, 682, 684) and wherein each of the second pairs of formations (110, 112, 499, 662, 664) comprises a pair of slots and wherein each pair of slots has a different length.

8. The trial implant system as claimed in claim 4, wherein the first pair of formations comprises a pair of slots and wherein each of the second pairs of formations comprises a pair of lugs and wherein each pair of lugs has a different length or a different position along the longitudinal axis.

9. The trial implant system as claimed in claim 1, and further comprising:
a second trial head having an articulating surface, the second trial head including a bore having a bore longitudinal axis and which can receive the free end of the neck with the neck longitudinal axis and bore longitudinal axis coincident, wherein the second trial head is a different size to the first trial head and includes either a further first formation or a further plurality of second formations angularly disposed around the longitudinal axis.

10. The trial implant system as claimed in claim 1, and further comprising:
a second trial head having an articulating surface, the second trial head including a bore having a bore longitudinal axis and which can receive the free end of the neck with the neck longitudinal axis and bore longitudinal axis coincident, wherein the second trial head is the same size as the first trial head and includes either a further first formation or a further plurality of second formations angularly disposed around the longitudinal axis and wherein the further first formation or the further plurality of second formations can limit the depth of insertion of the neck into the bore by different amounts which differ from those of the first trial head.

11. The trial implant system as claimed in claim 1, wherein the first trial head (100) includes a plurality of markings (116), each marking corresponding to a different off set associated with a respective one of the second formations.

12. The trial implant system as claimed in claim 11, wherein the plurality of markings (116) are provided on a side part of the articulating surface (102) and angularly disposed about the bore longitudinal axis.

13. The trial implant system as claimed in claim 1, wherein the neck (124) includes a plurality of markings (150), each marking corresponding to a different off set associated with a respective one of the second formations.

14. The trial implant system as claimed in claim 13, wherein the plurality of markings (150) are disposed at different positions along the neck longitudinal axis.

15. The trial implant system as claimed in claim 4, wherein each second pair of formations (110, 112, 210, 212, 499, 662, 664, 742, 744) is arranged in diametric opposition around the longitudinal axis.

## Patentansprüche

1. Test-Implantatsystem, umfassend:
eine Schaftkomponente (120, 220, 320, 680, 740) mit einem Hals (122, 222), welcher ein freies Ende (124, 224) enthält, wobei der Hals eine Hals-Längsachse (123) hat;
einen ersten Testkopf (100, 200, 490, 660, 720) mit einer Gelenkfläche (102), wobei der erste Testkopf eine Bohrung (108, 208, 498, 725) enthält, welche eine Bohrungslängsachse (106, 206) hat und welche das freie Ende des Halses aufnehmen kann, wobei die Hals-Längsachse und die Bohrungslängsachse zusammenfallen;
eine erste Ausbildung (136, 134, 234, 236, 504, 506, 566, 568, 588, 604, 606, 656, 682, 684, 722, 724); und
mehrere zweite Ausbildungen (110, 112, 210, 212, 499, 662, 664, 742, 744), wobei eine von erster Ausbildung und den mehreren zweiten Ausbildungen an dem Hals vorgesehen ist und die andere in der Bohrung vorgesehen ist und wobei jede der zweiten Ausbildungen mit der ersten Ausbildung passend in Eingriff treten kann, um die Einführtiefe des Halses in die Bohrung um einen unterschiedlichen Betrag zu begrenzen, **dadurch gekennzeichnet, dass** die mehreren zweiten Ausbildungen winkelig um die Längsachse herum angeordnet sind und die erste Ausbildung und die mehreren zweiten Ausbildungen gleitend in Eingriff kommen, wenn der erste Testkopf durch Verschieben entlang der Längsachse von der Schaftkomponente gelöst oder abgenommen wird.

2. Test-Implantatsystem nach Anspruch 1, wobei die erste Ausbildung (682, 684, 722, 724) und die mehreren zweiten Ausbildungen (662, 664, 742, 744) an dem Hals und in der Bohrung derart angeordnet sind, dass sie gleitend ausklinkbar sind, während ein Endabschnitt des freien Endes des Halses noch innerhalb der Bohrung des ersten Testkopfs angeordnet ist.

3. Test-Implantatsystem nach Anspruch 2, wobei die erste Ausbildung (682, 684, 722, 724) oder die mehreren zweiten Ausbildungen (662, 664, 742, 744) vor einer letzten Fläche des freien Endes des Halses (686) oder einer Mündung der Bohrung (726) des Testkopfs enden.

4. Test-Implantatsystem nach einem der vorhergehenden Ansprüche, wobei die erste Ausbildung (136, 134, 234, 236, 504, 506, 566, 568, 586, 588, 604, 606, 682, 684, 722, 724) ein erstes Paar von Ausbildungen umfasst und die mehreren zweiten Ausbildungen (110, 112, 210, 212, 499, 662, 664, 742, 744) mehrere zweite Paare von Ausbildungen umfassen.

5. Test-Implantatsystem nach einem der vorhergehenden Ansprüche, wobei die erste Ausbildung (134, 136, 504, 506, 566, 568, 586, 588, 604, 606, 656, 684, 682) an dem freien Ende des Halses angeordnet ist und die mehreren zweiten Ausbildungen (110, 112, 499, 662, 664) in der Bohrung des ersten Testkopfs angeordnet sind.

6. Test-Implantatsystem nach einem der Ansprüche 1 bis 4, wobei die erste Ausbildung (244, 246, 722, 724) in der Bohrung des ersten Testkopfs ist und die mehreren zweiten Ausbildungen (210, 212, 742, 744) an dem freien Ende des Halses angeordnet sind.

7. Test-Implantatsystem nach Anspruch 4, wobei das erste Paar von Ausbildungen ein Paar Ansätze (134, 136, 504, 506, 566, 568, 586, 588, 604, 606, 682, 684) umfasst, und wobei jedes der zweiten Paare von Ausbildungen (110, 112, 499, 662, 664) ein Paar Schlitze umfasst, und wobei jedes Paar Schlitze eine andere Länge hat.

8. Test-Implantatsystem nach Anspruch 4, wobei das erste Paar von Ausbildungen ein Paar Schlitze umfasst, und wobei jedes der zweiten Paare von Ausbildungen ein Paar Ansätze umfasst, und wobei jedes Paar Ansätze eine andere Länge oder eine andere Position entlang der Längsachse hat.

9. Test-Implantatsystem nach Anspruch 1, und ferner umfassend:
einen zweiten Testkopf mit einer Gelenkfläche, wobei der zweite Testkopf eine Bohrung enthält, welche eine Bohrungslängsachse hat und das freie Ende des Halses aufnehmen kann, wobei die Längsachse des Halses und die Bohrungslängsachse zusammenfallen, wobei der zweite Testkopf eine andere Größe als der erste Testkopf hat und entweder eine weitere erste Ausbildung oder weitere mehrere zweite Ausbildungen enthält, welche winklig um die Längsachse herum angeordnet sind.

10. Test-Implantatsystem nach Anspruch 1, und ferner umfassend:
einen zweiten Testkopf mit einer Gelenkfläche, wobei der zweite Testkopf eine Bohrung enthält, welche eine Bohrungslängsachse hat und das freie Ende des Halses aufnehmen kann, wobei die Längsachse des Halses und die Bohrungslängsachse zusammenfallen, wobei der zweite Testkopf die gleiche Größe wie der erste Testkopf hat und entweder eine weitere erste Ausbildung oder weitere mehrere zweite Ausbildungen enthält, welche winklig um die Längsachse herum angeordnet sind, und wobei die weitere erste Ausbildung oder die weiteren mehreren zweiten Ausbildungen die Einführtiefe des Halses in die Bohrung um verschiedene Beträge begrenzen können, die sich von denen des ersten Testkopfs unterscheiden.

11. Test-Implantatsystem nach Anspruch 1, wobei der erste Testkopf (100) mehrere Markierungen (116) enthält, wobei jede Markierung einem anderen Versatz entspricht, welcher einer entsprechenden der zweiten Ausbildungen zugehörig ist.

12. Test-Implantatsystem nach Anspruch 11, wobei die mehreren Markierungen (116) auf einem Seitenabschnitt der Gelenkfläche (102) vorgesehen sind und winklig um die Bohrungslängsachse herum angeordnet sind.

13. Test-Implantatsystem nach Anspruch 1, wobei der Hals (124) mehrere Markierungen (150) enthält, wobei jede Markierung einem anderen Versatz entspricht, welcher einer entsprechenden der zweiten Ausbildungen zugehörig ist.

14. Test-Implantatsystem nach Anspruch 13, wobei die mehreren Markierungen (150) an unterschiedlichen Positionen entlang der Längsachse des Halses angeordnet sind.

15. Test-Implantatsystem nach Anspruch 4, wobei jedes zweite Paar Ausbildungen (110, 112, 210, 212, 499, 662, 664, 742, 744) einander diametral gegenüber liegend um die Längsachse herum angeordnet ist.

## Revendications

1. Système d'implant d'essai comprenant :
un composant formant tige (120, 220, 320, 680, 740) ayant un col (122, 222) comprenant une extrémité libre (124, 224), le col ayant un axe longitudinal (123) de col ;
une première tête d'essai (100, 200, 490, 660, 720) ayant une face qui s'articule (102), la première tête d'essai comprenant un alésage (108, 208, 498, 725) ayant un axe longitudinal (106, 206) d'alésage et qui peut recevoir l'extrémité libre du col avec l'axe longitudinal du col et l'axe longitudinal de l'alésage qui coïncident ;
une première formation (136, 134, 234, 236, 504, 506, 566, 568, 586, 588, 604, 606, 656, 682, 684, 722, 724) ; et
une pluralité de secondes formations (110, 112, 210, 212, 499, 662, 664, 742, 744),
dans lequel une formation parmi la première formation et la pluralité de secondes formations est prévue sur le col et l'autre formation est prévue à l'intérieur de l'alésage, et chacune des secondes formations peut venir en prise de manière appariée avec la première [paire de] formation[s] afin de limiter la profondeur d'insertion du col à l'intérieur de l'alésage d'un degré différent, **caractérisé en ce que** la pluralité de secondes formations est disposée de manière angulaire autour de l'axe longitudinal et la première formation et la pluralité de secondes formations viennent en prise de manière coulissante lorsque la première tête d'essai est retirée ou détachée du composant formant tige par translation le long de l'axe longitudinal.

2. Système d'implant d'essai selon la revendication 1, dans lequel la première formation (682, 684, 722, 724) et la pluralité de secondes formations (662, 664, 742, 744) sont positionnées sur le col et à l'intérieur de l'alésage de manière à pouvoir se désolidariser de manière coulissante tandis que la partie terminale de l'extrémité libre du col reste à l'intérieur de l'alésage de la première tête d'essai.

3. Système d'implant d'essai selon la revendication 2, dans lequel la première formation (682, 684, 722, 724) ou la pluralité de secondes formations (662, 664, 742, 744) se terminent près de la face la plus distale de l'extrémité libre du col (686) ou près d'une ouverture de l'alésage (726) de la tête d'essai.

4. Système d'implant d'essai selon l'une quelconque des revendications précédentes, dans lequel la première formation (136, 134, 234, 236, 504, 506, 566, 568, 586, 588, 604, 606, 682, 684, 722, 724) comprend une première paire de formations et la pluralité de secondes formations (110, 112, 210, 212, 499, 662, 664, 742, 744) comprend une pluralité de secondes paires de formations.

5. Système d'implant d'essai selon l'une quelconque des revendications précédentes, dans lequel la première formation (134, 136, 504, 506, 566, 568, 586, 588, 604, 606, 656, 684, 682) est prévue sur l'extrémité libre du col et la pluralité de secondes formations (110, 112, 499, 662, 664) est prévue à l'intérieur de l'alésage de la première tête d'essai.

6. Système d'implant d'essai selon l'une quelconque des revendications 1 à 4, dans lequel la première formation (244, 246, 722, 724) est à l'intérieur de l'alésage de la première tête d'essai et la pluralité de secondes formations (210, 212, 742, 744) est prévue sur l'extrémité libre du col.

7. Système d'implant d'essai selon la revendication 4, dans lequel la première paire de formations comprend une paire d'ergots (134, 136, 504, 506, 566, 568, 586, 588, 604, 606, 682, 684) et dans lequel chacune des secondes paires de formations (110, 112, 499, 662, 664) comprend une paire de fentes, chaque paire de fentes étant de longueur différente.

8. Système d'implant d'essai selon la revendication 4, dans lequel la première paire de formations comprend une paire de fentes et dans lequel chacune des secondes paires de formations comprend une paire d'ergots, chaque paire d'ergots ayant une longueur différente ou une position différente le long de l'axe longitudinal.

9. Système d'implant d'essai selon la revendication 1, et comprenant en outre :
une seconde tête d'essai ayant une surface qui s'articule, la seconde tête d'essai comprenant un alésage ayant un axe longitudinal d'alésage et pouvant recevoir l'extrémité libre du col avec l'axe longitudinal du col et l'axe longitudinal de l'alésage qui coïncident, la seconde tête d'essai ayant une taille différente de celle de la première tête d'essai et comprenant soit une autre première formation, soit une autre pluralité de secondes formations disposées angulairement autour de l'axe longitudinal.

10. Système d'implant d'essai selon la revendication 1, et comprenant en outre :
une seconde tête d'essai ayant une surface qui s'articule, la seconde tête d'essai comprenant un alésage ayant un axe longitudinal d'alésage et pouvant recevoir l'extrémité libre du col avec l'axe longitudinal du col et l'axe longitudinal de l'alésage qui coïncident, la seconde tête d'essai présentant la même taille que celle de la première tête d'essai et comprenant soit une autre première formation, soit une autre pluralité de secondes formations disposées angulairement autour de l'axe longitudinal, et l'autre première formation ou l'autre pluralité de secondes formations pouvant limiter la profondeur d'insertion du col dans l'alésage de diverses valeurs différant de celles de la première tête d'essai.

11. Système d'implant d'essai selon la revendication 1, dans lequel la première tête d'essai (100) comprend une pluralité de repères (116), chaque repère correspondant à un décalage différent associé respectivement à une des secondes formations.

12. Système d'implant d'essai selon la revendication 11, dans lequel la pluralité de repères (116) est prévue sur une partie latérale de la face qui s'articule (102) et disposée angulairement autour de l'axe longitudinal de l'alésage.

13. Système d'implant d'essai selon la revendication 1, dans lequel le col (124) comprend une pluralité de repères (150), chaque repère correspondant à un décalage différent associé à une formation respective des secondes formations.

14. Système d'implant d'essai selon la revendication 13, dans lequel la pluralité de repères (150) est disposée à différentes positions le long de l'axe longitudinal du col.

15. Système d'implant d'essai selon la revendication 4, dans lequel chaque seconde paire de formations (110, 112, 210, 212, 499, 662, 664, 742, 744) est agencée de manière diamétralement opposée autour de l'axe longitudinal.
